# FASCICULE DE BREVET EUROPEEN

(11) **EP 2 605 781 B1**
(45) Date de publication et mention de la délivrance du brevet: **21.09.2016**
(21) Numéro de dépôt: 11758509.1
(22) Date de dépôt: 16.08.2011
(51) Int. Cl.: A61K 35/16

(54) **PROCÉDÉ DE LYOPHILISATION DE PLASMA SANGUIN**
GEFRIERTROCKNUNGSVERFAHREN FÜR BLUTPLASMA
BLOOD PLASMA LYOPHILIZATION PROCESS

(30) Priorité: 16.08.2010 FR 1056619
(43) Date de publication de la demande: 26.06.2013
(73) Titulaire: Etat Français (Ministère de la Défense), Service de Sante des Armees, 00457 Armees (FR)
(72) Inventeur: SAILLIOL, Anne, F-92290 Chatenay Malabry (FR)
(74) Mandataire: Cabinet Plasseraud
(86) Numéro de dépôt international: PCT/FR2011/051919
(87) Numéro de publication internationale: WO 2012/022914

(56) Documents cités:
- WO-A1-2005/058334
- US-A1- 2002 182 195
- "Freeze-dried plasma without blood group and its preparation method", DERWENT, 18 avril 2001 (2001-04-18), XP002280416,
- MUELLER MARKUS M ET AL: "Effects of pathogen inactivation using Amotosalen-UVA on coagulation parameters in apheresis and whole blood derived frozen plasma in a large blood bank setting.", BLOOD, vol. 108, no. 11, Part 1, novembre 2006 (2006-11), pages 281A-282A, XP009144715, & 48TH ANNUAL MEETING OF THE AMERICAN-SOCIETY-OF-HEMATOLOGY; ORLANDO, FL, USA; DECEMBER 09 -12, 2006 ISSN: 0006-4971

## Description

La présente invention concerne un procédé de préparation d'un plasma lyophilisé déleucocyté, viro-atténué, exempt d'anticorps hémolysant, et compatible avec tous les groupes sanguins.

Le "plasma sanguin" ou "plasma" est le composant liquide du sang, dans lequel les cellules sanguines sont en suspension. Le plasma est susceptible d'être séparé du sang total par centrifugation ou filtration à travers une membrane. Le plasma sanguin est constitué essentiellement d'eau, de protéines plasmatiques (principalement l'albumine et des anticorps) et des facteurs de coagulation dont le fibrinogène.

Le plasma sanguin est utilisé en thérapeutique pour le traitement de coagulopathies graves avec effondrement de tous les facteurs de coagulation ainsi que pour le traitement d'hémorragies aiguës, avec déficit global des facteurs de coagulation.

Actuellement, le plasma thérapeutique est du plasma frais congelé (PFC) viro-atténué. Un tel plasma nécessite une conservation à une température inférieure ou égale à - 25°C, ce qui implique que son administration ne peut s'effectuer qu'après décongélation dans des conditions spécifiques. De plus, ce type de plasma ne peut être conservé plus d'un an, ce qui génère des pertes conséquentes. Enfin, ce plasma thérapeutique doit être utilisé avec précaution puisque son administration doit respecter les règles de délivrance basées sur les compatibilités ABO des receveurs. Du fait des restrictions liées à son utilisation et à sa conservation et donc sa disponibilité, un tel plasma frais congelé s'avère inadapté à une application dans des conditions d'urgence, notamment lors d'actions militaires.

Il existe donc un besoin pour un plasma répondant aux contraintes opérationnelles des militaires sur les théâtres d'opération extérieurs, susceptible d'être conservé à température ambiante, exempt de toute forme de contamination bactérienne, virale ou parasitaire et compatible avec n'importe quel receveur, indépendamment de son groupe sanguin ABO.

A l'issue de recherches longues et approfondies, les inventeurs ont développé un procédé de préparation d'un plasma lyophilisé dont les caractéristiques répondent à de telles exigences de conservation et d'utilisation.

Ainsi l'invention concerne un procédé de préparation de plasma lyophilisé atténué comprenant les étapes suivantes:
a) la sélection de plasmas unitaires déleucocytés et atténués physico-chimiquement;
b) le mélange des plasmas unitaires sélectionnés; et
c) la lyophilisation dudit mélange de plasmas.

Le procédé de l'invention permet *in fine* l'obtention d'un "plasma lyophilisé" ou "plasma cryodesséché", grâce à une étape de lyophilisation appropriée. Le plasma obtenu après reconstitution du plasma lyophilisé dans un solvant de reprise, répond aux exigences réglementaires auxquelles sont soumis les plasmas actuellement utilisés en thérapeutique, en particulier les concentrations en facteurs de coagulation sont satisfaisantes (par exemple la concentration en facteur VIII est supérieure ou égale à 0,5 UI/mL) et il n'y a pas d'activation de la coagulation.

Par "plasma unitaire", on entend du plasma recueilli à partir d'un seul individu donneur. Ce plasma unitaire peut être préparé à partir du sang total ou être recueilli par aphérèse. Préférentiellement, dans le cadre de cette invention, les plasmas unitaires constituant le mélange de plasmas de l'étape b) du procédé de l'invention sont recueillis par aphérèse. Ce plasma unitaire est placé dans une poche de plasma. Généralement ces poches contiennent entre 200 et 250 mL de plasma unitaire. Préférentiellement, les plasmas unitaires constituant le mélange de plasmas de l'étape b) répondent aux mêmes exigences réglementaires auxquelles sont soumis les plasmas actuellement utilisés en thérapeutique. Ils respectent donc les exigences en vigueur, notamment en termes de taux de facteurs d'hémostase. Les individus donneurs doivent donc respecter les critères réglementaires d'éligibilité au don du plasma. Préférentiellement, les plasmas unitaires sont obtenus à partir de donneurs masculins ou exempts d'anticorps anti-HLA. En outre, dans le contexte de la présente invention, les donneurs doivent présenter un bilan d'hémostase normal et caractérisé par un taux de facteur VIII au moins égal à 0,9 UI/mL.

Par "individu donneur", on entend un individu susceptible de faire don de son sang ou des composants sanguins.

Par "mélange de plasmas" ou "lot transfusionnel", on entend un mélange de plasmas unitaires.

Par "sang total", on entend l'ensemble des composés et des cellules constituant le sang.

Par "aphérèse", on entend une technique de prélèvement, chez un donneur, de certains composants sanguins. Les composants que l'on souhaite prélever sont séparés par centrifugation et stockés, tandis que les composants non prélevés sont réinjectés au donneur.

Dans un mode de réalisation particulier, les plasmas unitaires constituant le mélange de plasmas de l'étape b) du procédé selon l'invention sont obtenus à partir d'au plus dix individus donneurs différents. Une telle limitation quant au nombre d'individu donneurs permet de réduire considérablement le risque infectieux résiduel tout en bénéficiant des avantages du mélange : immunogénicité réduite et obtention d'un plasma universel pour le groupage sanguin. Il permet en outre une traçabilité simplifiée des individus donneurs.

Il est également décrit ici un mode de réalisation dans lequel les plasmas unitaires constituant le mélange de plasmas de l'étape b) du procédé selon l'invention sont obtenus à partir d'au plus vingt individus donneurs différents.

Préférentiellement, lesdits individus donneurs appartiennent au groupe sanguin AB. Ainsi, la sélection des plasmas unitaires s'effectue de telle sorte que chacun des plasmas utilisés correspond à des plasmas obtenus à partir d'individus donneurs appartenant au groupe sanguin AB. Les plasmas unitaires obtenus à partir d'individus donneurs appartenant à ce groupe sont caractérisés par une absence d'agglutinines anti-A et anti-B, ce qui permet l'administration de leur plasma indépendamment du groupe sanguin ABO du receveur. Cette situation idéale est rarement possible en raison de la rareté des individus donneurs appartenant au groupe sanguin AB. Aussi, dans un mode de réalisation particulier de l'invention, lesdits individus donneurs sont caractérisés en ce qu'au moins un desdits individus donneurs appartient au groupe sanguin A et qu'au moins un desdits individus donneurs appartient au groupe sanguin B et en ce que le volume de plasma obtenu à partir du ou des individu(s) donneur(s) appartenant au groupe sanguin A est identique au volume de plasma obtenu à partir du ou des individu(s) donneur(s) appartenant au groupe sanguin B.

Ainsi, la sélection des plasmas unitaires peut s'effectuer de telle sorte que parmi les individus donneurs des plasmas unitaires, certains appartiennent au groupe sanguin A, d'autres au groupe sanguin B et éventuellement d'autres au groupe sanguin AB. Dans tous les cas, le volume de plasma obtenu à partir des individus donneurs appartenant au groupe sanguin A est identique au volume de plasma obtenu à partir des individus donneurs appartenant au groupe sanguin B.

Par exemple, les plasmas unitaires constituant le mélange de plasmas de l'étape b) du procédé peuvent être recueillis chez des individus donneurs dont les groupes sanguins sont les suivantes :
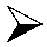 4 individus donneurs appartenant au groupe sanguin A;
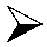 4 individus donneurs appartenant au groupe sanguin B; et
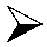 2 individus donneurs appartenant au groupe sanguin AB.

Dans ce cas particulier, il convient de veiller à ce que le volume de plasma obtenu à partir des 4 individus donneurs appartenant au groupe sanguin A soit identique au volume de plasma obtenu à partir des 4 individus appartenant au groupe sanguin B.

Ainsi, grâce à la sélection des plasmas unitaires, l'invention pallie à l'inconvénient lié au nombre réduit d'individus donneurs appartenant au groupe AB, dits "donneurs universels de plasma", pour l'obtention d'un plasma administrable chez n'importe quel individu receveur, indépendamment de son groupe sanguin ABO.

Dans un mode de réalisation particulier, le mélange de plasmas de l'étape b) du procédé comprend :
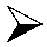 20 à 50%, préférentiellement 30 à 50%, plus préférentiellement 40 à 45% en volume de plasmas unitaires obtenus à partir d'individus donneurs appartenant au groupe sanguin A;
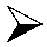 20 à 50%, préférentiellement 30 à 50%, plus préférentiellement 40 à 45 % en volume de plasmas unitaires obtenus à partir d'individus donneurs appartenant au groupe sanguin B; et
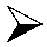 0 à 60 %; préférentiellement 0 à 40%, plus préférentiellement 10 à 20% en volume de plasmas unitaires obtenus à partir d'individus donneurs appartenant au groupe sanguin AB.

Par "groupe sanguin", on entend une classification de sang reposant sur la présence ou l'absence de substances antigéniques sur la surface des hématies. Ces substances antigéniques définissent le système ABO. L'antigène A, ou agglutinogène A, correspond à une N-acétyl-galactosamine. L'antigène B, ou agglutinogène B, correspond à un galactose. Le système ABO dicte les règles de compatibilité de la transfusion sanguine. Le non respect de ces règles peut entrainer un accident hémolytique chez l'individu transfusé. Comme le plasma contient des anticorps en fonction du groupe dans le système ABO, les globules rouges du receveur ne doivent pas présenter les antigènes correspondants. Ainsi, il convient de ne pas administrer un plasma comprenant des agglutinines anti-A à un patient appartenant au groupe sanguin A, et *vice versa.* Le plasma d'individus donneurs appartenant au groupe AB ne contient pas d'agglutinines anti-A ou anti-B et convient ainsi à tous les individus receveurs. On parle donc de "donneurs universels de plasma".

Par "individu appartenant au groupe sanguin A, B ou AB", on entend un individu possédant respectivement le phénotype A, B ou AB.

Les plasmas unitaires constituant le mélange de plasmas de l'étape b) du procédé de l'invention sont déleucocytés. La présence de leucocytes dans les produits transfusés peut causer des effets indésirables divers tels que la transmission de virus comme le cytomégalovirus. La déleucocytation permet également la réduction des réactions fébriles liées à des réactions antigènes-anticorps dans le système HLA. Cette déleucocytation, également appelée "réduction de leucocytes" ou "réduction leucocytaire" s'effectue par filtration sur le plasma recueilli par aphérèse. Alternativement, cette déleucocytation peut s'effectuer par filtration sur le plasma préparé à partir de sang total.

Préférentiellement, les plasmas unitaires constituant le mélange de plasmas de l'étape b) sont exempts d'anticorps hémolysant. La vérification de l'absence d'anticorps hémolysant s'effectue directement sur les plasmas unitaires recueillis chez l'individu donneur. Ainsi, l'absence d'anticorps hémolysant dans les plasmas recueillis permet l'obtention d'un mélange de plasmas à l'étape b) ne contenant aucun anticorps hémolysant.

Les "anticorps hémolysants" ou "hémolysines" sont des immunoglobulines G ou IgG que l'on peut trouver dans le plasma et qui sont susceptibles de lyser les hématies. Les hémolysines anti-A sont spécifiques des hématies présentant l'agglutinogène A, tandis que les hémolysines anti-B sont spécifiques des hématies présentant l'agglutinogène B. Leur présence dans un plasma thérapeutique « universel » est inacceptable. La présence de tels anticorps hémolysants dans le mélange de plasmas rendrait le produit obtenu par le procédé inadapté à une utilisation « universelle » en thérapeutique. Aussi, les plasmas unitaires constituant le mélange de plasmas de l'étape b) du procédé de l'invention ne doivent pas contenir des hémolysines anti-A ou anti-B.

Préférentiellement, les plasmas unitaires constituant le mélange de plasmas de l'étape b) sont exempts d'agglutinine. Typiquement, les plasmas ne comprenant pas d'agglutinines sont des plasmas obtenus à partir d'individus appartenant au groupe sanguin AB.

Alternativement, les plasmas unitaires constituant le mélange de plasmas de l'étape b) présentent un titre en agglutinines inférieur à 64, préférentiellement inférieur à 32, préférentiellement inférieur à 16, préférentiellement inférieur à 8, plus préférentiellement inférieur à 4. La détermination du titre en anticorps est bien connue de l'homme du métier. Il s'agit d'établir une série de dilutions du plasma et d'effectuer la réaction de détection d''agglutinines avec chaque dilution. La plus haute dilution offrant encore une réaction positive donnera le titre. Ainsi, lorsqu'à une dilution de 1/32 on détecte encore les agglutinines à titrer, mais que cette détection ne s'effectue plus à une dilution de 1/64, le titre en agglutinine dans ce plasma est de 32.

Les agglutinines sont des immunoglobulines IgM présentes dans le plasma et susceptibles d'agglutiner les hématies. Les agglutinines anti-A agglutinent les hématies présentant l'agglutinogène A. Les agglutinines anti-B agglutinent les hématies présentant l'agglutinogène B. L'absence d'agglutinines ou sa présence à une concentration faible dans le mélange de plasmas obtenu à l'étape b) permet *in fine* l'obtention d'un plasma lyophilisé selon le procédé de l'invention qui pourra être utilisé, après reconstitution, sur n'importe quel receveur, indépendamment de son groupe sanguin.

Préférentiellement, le mélange de plasmas de l'étape b) du procédé est exempt d'agglutinine irrégulière. Les agglutinines irrégulières sont des anticorps susceptibles d'être présents dans le plasma et qui sont dirigés contre des antigènes présents sur la surface des hématies mais ne correspondant pas aux agglutinogènes A ou B.

Les plasmas unitaires constituant le mélange de plasmas de l'étape b) du procédé de l'invention sont viro-atténués ou sécurisés par traitement physico-chimique.

Par "atténuation", "viro-atténuation" ou "sécurisation", on entend l'élimination d'agents pathogènes susceptibles d'être présent dans le plasma. Cette viro-atténuation ou sécurisation détruit la majorité des pathogènes enveloppés ou non-enveloppés ou empêche leur réplication.

Dans le contexte de cette invention, l'atténuation se fait par traitement physico-chimique des plasmas unitaires, préalablement à leur mélange. Alternativement, cette atténuation peut se faire sur le mélange de plasmas obtenu à l'étape b). L'atténuation par traitement physico-chimique peut être un traitement à l'aide d'un agent photochimique tel que l'amotosalen ou le bleu de méthylène, ou par solvant-détergeant.

Préférentiellement, cette atténuation s'effectue à l'aide d'un agent photochimique tel que l'amotosalen, le bleu de méthylène ou la riboflavine.

Par "agent pathogène", on entend un contaminant bactérien, viral ou parasitaire. La présence de tels contaminants est inacceptable pour l'utilisation d'un plasma en thérapeutique.

Par "plasma atténué" ou "plasma viro-atténué" on entend un plasma ayant subi une étape d'atténuation, c'est-à-dire la destruction réelle ou l'inhibition de la réplication d'agents pathogènes tels que des contaminants bactériens, viraux ou parasitaires.

Dans un mode de réalisation particulier, les plasmas unitaires constituant le mélange de plasmas de l'étape b) du procédé de l'invention sont atténués par action d'un agent photochimique choisi parmi l'amotosalen, la riboflavine et le bleu de méthylène. L'utilisation d'un tel agent photochimique implique l'exposition à une source lumineuse. Quand il est activé par la lumière d'une longueur d'onde appropriée, l'agent photochimique, également appelé agent photo-oxydant, peut détruire directement le contaminant bactérien ou viral ou bien inhiber sa capacité à se répliquer. Une telle technique d'atténuation du plasma est particulièrement avantageuse puisqu'elle permet la destruction de tout contaminant, y compris ceux qui ne sont pas détectables par les techniques classiques de l'art antérieur. Les conditions opératoires permettant l'élimination d'agents pathogènes à l'aide d'un agent photochimique sont connues de l'homme du métier. Typiquement, cette méthode est réalisée suivant un protocole standardisé basé sur l'utilisation d'un dispositif à usage unique. Le plasma est additionné de l'agent photochimique. Le plasma est ensuite soumis à une source lumineuse. La dernière étape consiste à éliminer les traces de l'agent photochimique résiduel et ses éventuels produits de dégradation à l'aide d'un filtre permettant leur absorption. Selon l'invention, cette technique d'élimination d'agents pathogènes est préférentiellement appliquée à chaque plasma unitaire recueilli par aphérèse.

Préférentiellement, cet agent photochimique est l'amotosalen. L'élimination d'agents pathogènes par amotosalen présente l'intérêt de ne pas entrainer une perte trop importante des facteurs de coagulation présents dans le plasma, et tout particulièrement le fibrinogène (qui joue un rôle important pour l'hémostase du traumatisé hémorragique) et le facteur VIII. L'amotosalen est un dérivé des psoralènes qui s'intercale de façon réversible au niveau des bases pyrimidiques des molécules d'ADN ou d'ARN, simple ou double brin. L'illumination par la lumière ultraviolette A (380 à 400 nm) crée des liaisons covalentes irréversibles qui interrompent les acides nucléiques et bloquent leur réplication, permettant ainsi de réduire la charge bactérienne, virale ou parasitaire et d'inhiber la réplication bactérienne, virale ou parasitaire dans le plasma avant lyophilisation. Cet agent est particulièrement efficace pour l'inactivation de virus enveloppés ou non, de bactéries Gram + et Gram -, de spirochètes, de spores, de parasites et de lymphocytes résiduels. Diverses études ont montré que cet agent ne présente pas de risque de toxicité à long terme ni de toxicité reproductive. Il n'est pas carcinogène et ne présente aucun effet toxique notable. Typiquement, pour que l'élimination d'agents pathogènes soit efficace, il convient de traiter les plasmas unitaires avec de l'amotosalen à une concentration comprise entre 100 et 200 µM, préférentiellement environ 150 µM. Après traitement des plasmas unitaires, il convient d'éliminer les traces d'amotosalen résiduelles. L'homme du métier peut mettre en oeuvre des tests de contrôle pour détecter la présence d'amotosalen résiduel. Cette concentration en amotosalen résiduel ne doit préférentiellement pas excéder 2 µM.

Préférentiellement, cet agent photochimique est la riboflavine. La riboflavine, ou vitamine B2, est un composé naturel, non toxique, qui lorsqu'il est utilisé en combinaisons avec les rayonnements ultraviolets permet l'inactivation des virus, des bactéries et des parasites. Son utilisation permet en outre la neutralisation des globules blancs présents dans certains constituants du sang. Typiquement, la riboflavine est utilisée à l'aide du dispositif Mirasol, commercialisé pour la société CaradianBCT. Il est actuellement utilisé pour le traitement de plaquettes en suspension dans du plasma, et pour le traitement de plasma frais congelé. Les inventeurs ont montré que ce système est également hautement adapté pour une utilisation dans le contexte de la présente invention.

Les plasmas unitaires déleucocytés et atténués peuvent être surgelés dans les 8 heures qui suivent le prélèvement chez les individus donneurs. Les plasmas ainsi congelés sont ensuite conservés à une température inférieure ou égale à -25°C jusqu'à la préparation du mélange de ces plasmas. Ces plasmas sont ensuite décongelés avant de procéder à leur mélange. Typiquement, les plasmas unitaires sont décongelés dans un bain marie à 37°C. Ainsi, l'étape a) peut être réalisée de la façon suivante:
i. collecte de plasmas unitaires par aphérèse;
ii. déleucocytation desdits plasmas unitaires;
iii. atténuation physico-chimique desdits plasmas unitaires;
iv. surgélation desdits plasmas unitaires dans les 8 heures suivant la collecte des plasmas unitaires;
v. conservation desdits plasmas unitaires congelés à une température inférieure ou égale à -25°C;
vi. décongélation des plasmas unitaires congelés, préférentiellement dans un bain marie à 37°C pendant une durée inférieure à 30 minutes, préférentiellement pour une durée de 15 minutes environ.

Selon un mode de réalisation particulier, l'invention concerne un procédé de préparation de plasma lyophilisé atténué comprenant les étapes suivantes:
a) la sélection de plasmas unitaires déleucocytés et atténués physico-chimiquement par:
   i.collecte de plasmas unitaires par aphérèse,
   ii.déleucocytation desdits plasmas unitaires,
   iii.atténuation physico-chimique desdits plasmas unitaires,
   iv.surgélation desdits plasmas unitaires dans les 8 heures suivant la collecte des plasmas unitaires,
   v.conservation desdits plasmas unitaires congelés à une température inférieure ou égale à -25°C,
   vi.décongélation des plasmas unitaires congelés;
b) le mélange des plasmas unitaires sélectionnés; et
c) la lyophilisation dudit mélange de plasmas.

Dans un mode de réalisation particulier, l'invention concerne un procédé de préparation de plasma lyophilisé sécurisé comprenant les étapes suivantes:
A) la sélection de plasmas unitaires déleucocytés et sécurisé par amotosalen et provenant de donneurs présentant un bilan de coagulation normal et un taux en facteur VIII supérieur à 0,9 UI/mL, lesdits plasmas étant obtenus à partir d'au plus dix individus donneurs différents et lesdits donneurs étant choisis parmi les donneurs masculins ou les donneurs exempts d'anticorps anti-HLA;
B) le mélange des plasmas unitaires sélectionnés; et
C) la lyophilisation dudit mélange de plasmas.

Le procédé de l'invention comprend une étape de mélange des plasmas unitaires. On obtient alors un "lot transfusionnel". Le mélange des plasmas unitaires s'effectue à température ambiante. Typiquement, on mélange les plasmas unitaires (contenus dans les poches de plasmas) pour obtenir un lot transfusionnel de volume compris entre 2000 et 10000 mL, préférentiellement entre 2500 et 9000 mL. Dans un premier mode de réalisation, ce lot transfusionnel a un volume compris entre 2500 et 4000 mL, de préférence environ 3000 mL. Dans un second mode de réalisation, ce lot transfusionnel a un volume compris entre environ 5000 et environ 9000 mL, de préférence environ 6000 mL. On obtient ainsi un mélange de plasmas unitaires dont l'administration peut se faire indépendamment du groupe sanguin de l'individu donneur.

Typiquement, on répartit le contenu de ce lot transfusionnel dans des flacons en verre de perfusion de 500 mL. Cette répartition se fait dans des conditions stériles. Chaque flacon contient alors une quantité de plasmas de 200 à 250 mL, préférentiellement de 215 mL. Ces flacons, qu'on appelle "de type I", sont caractérisés en ce qu'ils sont neutres et n'interagissent pas avec leur contenu. Ainsi, aucune réaction n'a lieu entre le flacon et le plasma qu'il contient. Ce type de flacon est adapté à la préparation de produit injectable. Ces flacons répondent aux exigences de la Pharmacopée et sont aisément accessibles dans le commerce.

Le procédé de l'invention comporte une étape c) de lyophilisation du mélange de plasmas atténués. Cette étape de lyophilisation est particulièrement délicate puisqu'il convient de ne pas compromettre les propriétés hémostatiques du mélange de plasmas atténués. Il convient alors de maitriser l'équilibre entre un taux d'humidité résiduelle très faible et la conservation des facteurs de coagulation, qui peuvent s'avérer particulièrement sensible au procédé agressif de lyophilisation. Typiquement, cette étape de lyophilisation s'effectue sur les flacons de type I d'une capacité de 500 mL contenant les mélanges de plasmas tels qu'obtenus précédemment. Typiquement, chaque flacon contenant environ 215 mL de plasmas, est placé sur une étagère du lyophilisateur.

Préférentiellement, l'étape c) de lyophilisation permet l'obtention d'un plasma lyophilisé présentant un taux d'humidité inférieur à 2%, préférentiellement inférieur à 1%.

Typiquement, la lyophilisation de l'étape c) comprend plusieurs phases: congélation, dessiccation primaire ou sublimation, et dessiccation secondaire ou séchage final.

Par "lyophilisation" ou "cryodessication", on entend une opération de déshydratation à basse température qui consiste à éliminer par sublimation, la majeure partie de l'eau contenue dans un produit. Elle autorise une conservation à long terme grâce à l'abaissement de l'activité de l'eau du produit. Préférentiellement, dans le contexte de cette invention, la lyophilisation comporte une phase de congélation rapide à -50°C dans laquelle l'eau contenue dans le plasma est solidifiée. Typiquement, cette étape de congélation s'effectue avec une rampe d'une durée comprise entre 15 minutes et 60 minutes, préférentiellement d'environ 30 minutes et un palier d'une durée comprise entre 100 et 600 minutes, préférentiellement d'environ 300 minutes.

Ensuite, intervient la phase de sublimation, également appelée dessiccation primaire, qui va entrainer le passage de l'eau de la forme solide à la forme vapeur, sans passage par une forme liquide. Cette étape se fait sous une pression inférieure à 300 µBar et à une température comprise entre 10 et 15°C. Typiquement, le premier palier à 10°C a une rampe d'une durée comprise entre 20 et 120 minutes, préférentiellement d'environ 60 minutes et un palier d'une durée comprise entre 2000 et 4000 minutes, préférentiellement d'environ 3000 minutes. Le second palier à 15°C a une rampe d'une durée comprise entre 5 et 60 minutes, préférentiellement d'environ 10 minutes et un palier d'une durée comprise entre 800 et 2000 minutes, préférentiellement d'environ 1200 minutes.

La dernière étape, communément appelée "séchage final" ou "dessiccation secondaire", est l'étape qui consiste à enlever l'eau, dite captive, du produit par désorption. L'eau captive correspond aux molécules d'eau restant piégées en surface d'un produit soumis à une dessiccation primaire. Cette étape de séchage final s'effectue à une température comprise entre 30 et 35°C sous une pression réduite de 30 µBar environ. Typiquement, le premier palier à 35°C a une rampe d'une durée comprise entre 2000 et 15000 minutes, préférentiellement 6000 minutes et un palier d'une durée comprise entre 800 et 2000 minutes, préférentiellement 1200 minutes. Le second palier à 30°C a une rampe d'une durée comprise entre 2000 et 1000 minutes, préférentiellement 480 minutes et un palier d'une durée comprise entre 1200 et 2500 minutes, préférentiellement 1800 minutes.

Ce protocole de lyophilisation et ses conditions particulières permettent l'obtention d'un plasma lyophilisé présentant un taux d'humidité inférieur à 2%, préférentiellement inférieur à 1%.

Il est également décrit ici un plasma lyophilisé et compatible avec tous les groupes sanguins. De préférence, ce plasma lyophilisé est déleucocyté, atténué et exempt d'anticorps hémolysant pour répondre aux exigences réglementaires. On parle de "Plasma Cryodesséché Sécurisé " (PCSD) ou " plasma lyophilisé" (FLYP : "French Lyophilised Plasma ").

Typiquement, ce plasma lyophilisé comprend un mélange de plasmas recueillis à partir d'individus donneurs dont au moins un appartient au groupe sanguin A et au moins un appartient au groupe sanguin B et en ce que le volume de plasma obtenu à partir du ou des individu(s) donneur(s) appartenant au groupe sanguin A est identique au volume de plasma obtenu à partir du ou des individu(s) donneur(s) appartenant au groupe sanguin B.

Ce plasma lyophilisé présente un taux d'humidité inférieur à 2%, préférentiellement inférieur à 1%. Il est en outre caractérisé en ce qu'il peut être stocké à température ambiante ou dans une enceinte réfrigérée à une température comprise entre 2°C et 25°C et pour une durée de trois ans, préférentiellement de deux ans.

Préférentiellement, ce plasma sanguin lyophilisé est stérile. Il est également décrit ici un procédé de préparation de plasma reconstitué comprenant l'étape de reconstitution du plasma lyophilisé déleucocyté, atténué, exempt d'anticorps hémolysant, et compatible avec tous les groupes sanguins dans un solvant de reprise. La reconstitution du plasma permet ainsi l'obtention d'une préparation injectable, susceptible d'être administrée à n'importe quel receveur dans des conditions d'urgence.

Typiquement, la reconstitution du plasma s'effectue dans un volume de solvant de reprise compris entre 100 et 400 mL, préférentiellement de 200 mL. Typiquement, cette reconstitution s'effectue avec un volume permettant d'obtenir un plasma iso-osmotique.

Préférentiellement, ce solvant de reprise est l'eau et plus préférentiellement de l'eau pour préparation injectable. Préférentiellement, la reconstitution du plasma lyophilisé pour l'obtention d'une préparation injectable s'effectue en une durée inférieure à 6 minutes, préférentiellement inférieure à 3 minutes.

Aussi, l'utilisation du plasma décrit ici est très avantageuse et s'affranchit du temps nécessaire à la décongélation lors de l'utilisation de plasma frais congelé.

Il est également décrit ici un plasma reconstitué, déleucocyté, atténué, exempt d'anticorps hémolysant, et compatible avec tous les groupes sanguins. Un tel plasma reconstitué est susceptible d'être administré à n'importe quel individu, indépendamment de son groupe sanguin. Il est donc hautement adapté pour une utilisation dans des conditions d'urgence, notamment sur le terrain d'actions militaires mais aussi en secteur civil pour le traitement des urgences hémorragiques avec coagulopathie, notamment en situation isolée avec des conditions logistiques ne permettant pas de maîtriser une chaîne du froid négative. Le plasma reconstitué selon l'invention présente en outre pour avantage la destruction de la plupart des agents pathogènes ce qui réduit considérablement la diffusion potentielle de pathogènes aux individus receveurs. Ce plasma reconstitué répond à toutes les exigences réglementaires auxquelles sont soumis les plasmas utilisés en thérapeutique.

Préférentiellement, le plasma reconstitué décrit ici est exempt d'anticorps hémolysant.

Préférentiellement, le plasma reconstitué décrit ici est exempt d'agglutinine. Alternativement, le plasma reconstitué selon l'invention présente un titre en agglutinines inférieur à 64, préférentiellement inférieur à 32, préférentiellement inférieur à 16, préférentiellement inférieur à 8, et plus préférentiellement inférieur à 4.

Préférentiellement, le plasma reconstitué décrit ici est exempt d'agglutinine irrégulière.

Le plasma reconstitué de l'invention est caractérisé en ce que la concentration en facteur VIII est supérieure à 0,2 UI/mL, préférentiellement supérieure à 0,5 UI/mL, préférentiellement supérieure à 0,7 UI/mL, plus préférentiellement supérieure à 0,9 UI/mL et encore plus préférentiellement compris entre 0,5 et 1.5 UI/mL.

Le plasma reconstitué décrit ici est caractérisé en ce que la concentration en facteur V est supérieure à 0, 15 UI/mL, et préférentiellement comprise entre 0,7 et 1,2 UI/mL. Les unités internationales (UI) pour les facteurs de coagulation expriment l'activité plasmatique des protéines auxquelles cette expression est appliquée. Une unité internationale (UI) de ces protéines plasmatiques correspond à la quantité de ce facteur contenue dans un mL de plasma humain normal.

Le plasma reconstitué décrit ici est caractérisé en ce que la concentration en fibrinogène est supérieure à 1 g/L et plus préférentiellement comprise entre 2 et 4 g/L.

Le plasma reconstitué décrit ici est caractérisé en ce qu'il est stérile et apyrogène.

Enfinil est décrit ici un kit comprenant:
a) du plasma lyophilisé selon l'invention;
b) une quantité adaptée de solvant de reprise, préférentiellement de l'eau pour préparation injectable; et
c) une notice explicative.

Dans un mode de réalisation particulier, ledit kit comprend en outre:
d) une fiche de suivi clinique et biologique entrant dans le cadre d'une hémovigilance active ; et
e) une fiche technique de mise en oeuvre de la traçabilité du plasma selon l'invention.

Préférentiellement, ce plasma lyophilisé se présente sous une forme adaptée à son utilisation sur le théâtre des actions militaires et des environnements très isolés avec des difficultés logistiques pour maitriser une chaîne du froid négative.

### LEGENDE DES FIGURES

**Figure 1****:** Analyse du taux d'hémoglobine, de plaquette et de fibrinogène, avant et après perfusion de plasma lyophilisé reconstitué selon l'invention chez des soldats sur le terrain d'actions militaires.
   *Ns: signifie non significatif*
**Figure 2****:** Analyse du taux de prothrombine (TP) avant et après perfusion de plasma lyophilisé reconstitué selon l'invention.

### EXEMPLES

### Exemple 1 Lyophilisation d'un mélange de plasmas

### a) Sélection de plasmas unitaires

On recueille par aphérèse environ 200 mL du plasma à partir d'individus donneurs appartenant aux groupes sanguins A, B et AB.

Chaque individu donneur peut faire don de 2 ou 3 poches de plasmas à chaque don mais il peut entrer plus d'une fois dans la constitution du mélange. Ainsi chaque donneur peut donner d'environ 400 à environ 1800 mL de plasma.

Lors de la collecte du plasma, on procède à la déleucocytation desdits plasmas unitaires par filtration.

On procède ensuite à l'atténuation desdits plasmas unitaires à l'aide de l'amotosalen. Pour ce faire, chacune des poches de plasma unitaire est additionnée de 15 mL d'amotosalen à une concentration de 150µM. Les plasmas comprenant l'amotosalen subissent une illumination à la lumière ultraviolette A (380 à 400 nm) pendant 5 à 10 minutes. Après traitement, l'amotosalen est éliminée par filtration sur une résine absorbante.

On procède ensuite à la surgélation desdits plasmas unitaires atténués dans les 8 heures suivant la collecte des plasmas. Cette étape permet alors la conservation desdits plasmas à une température de -25°C.

15 minutes avant de procéder au mélange des plasmas, on place les plasmas à décongeler dans un bain marie à 37°C.

### b) Mélanges des plasmas unitaires sélectionnés

On prépare 6 mélanges de plasmas M1 à M6, également appelés lots transfusionnels. Chacun des mélanges M1 à M6 correspond à un mélange de plasmas obtenus à partir d'individus appartenant aux groupes sanguins A, B et AB. Par exemple, pour le mélange M1, on utilise :
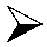 6 poches de plasmas (1327 mL de plasma) obtenus à partir de 3 individus donneurs différents appartenant au groupe sanguin A,
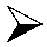 6 poches de plasmas (1339 mL de plasma) obtenus à partir de 3 individus donneurs différents appartenant au groupe sanguin B, et
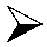 2 poches de plasmas (445 mL de plasma) obtenus à partir de 2 individus donneurs différents appartenant au groupe sanguin AB.

On obtient ainsi un mélange M1 comprenant 3111 mL de plasmas.

Le volume des différents plasmas utilisés pour ces mélanges et la répartition du groupe sanguin des individus donneurs pour chacun des mélanges M1 à M6 sont détaillés dans le tableau ci-après:

**Tableau 1 : Répartition du groupe sanguin des individus donneurs**

| **Mélange** | **Volume de plasma obtenu à partir de donneur de phénotype A (mL)** | **Volume de plasma obtenu à partir de donneur de phénotype B (mL)** | **Volume de plasma obtenu à partir de donneur de phénotype AB (mL)** |
|---|---|---|---|
| **M1** | 1327 | 1339 | 445 |
| **M2** | 1329 | 1336 | 445 |
| **M3** | 1346 | 1352 | 437 |
| **M4** | 1264 | 1258 | 638 |
| **M5** | 1327 | 1338 | 442 |
| **M6** | 1315 | 1327 | 442 |

On obtient ainsi 6 lots transfusionnels comprenant de 3000 à 3300 mL de plasmas.

### c) Etape de lyophilisation

Les mélanges de plasmas sont répartis dans des flacons de 500 mL "de type I", de telle sorte que chacun des flacons contienne 215 mL du mélange de plasmas.

La lyophilisation des plasmas contenus dans chacun des flacons précédemment obtenus se fait dans un lyophilisateur de type SMH 615, commercialisé par USIFROID. Chaque flacon est placé sur une étagère. La lyophilisation se fait dans les conditions particulières détaillées ci-après.

### 1. Pré-refroidissement

Cette étape permet le refroidissement des étagères du lyophilisateur à une température de -5°C. Cette étape permet d'éviter la dégradation des facteurs de coagulation qui sont thermosensibles pendant le temps de la répartition. Les lots sont chargés au fur et à mesure dans le lyophilisateur.

### 2. Congélation

Le mélange de plasmas est congelé à une température de -50°C. On maintient le produit à cette température pendant 240 minutes. La durée de la rampe est de 30 minutes et le palier est de 300 minutes.

### 3. Mise sous vide

Pour permettre la sublimation, on procédé à une mise sous vide du lyophilisateur. Le vide s'effectue pendant 2 minutes à une pression de 600 mBar.

### 4. Sublimation

Cette étape s'effectue à une température comprise entre 10 et 15°C et à une pression inférieure à 300 µBar.

Le premier palier à 10 °C de température a une rampe de 60 minutes et un palier de 3000 minutes.

Le second palier avec une température de 15 °C a une rampe de 10 minutes et un palier de 1200 minutes.

### 5. Dessication secondaire

Cette étape s'effectue à une température s'effectue à une température comprise entre 30 et 35°C sous une pression de 30 µBar.

Le premier palier à 35 °C a une rampe de 600 minutes et un palier de 1200 minutes.

Le second palier à 30 °C a une rampe de 480 minutes et un palier de 1800 minutes.

### d) Contrôles de la qualité du lyophilisat

Ce protocole permet l'obtention d'un plasma lyophilisé présentant un taux d'humidité relative inférieure à 2%.

### Exemple 2: Reconstitution des plasmas lyophilisés

On prend un flacon de 500 mL de chacun des lots transfusionnels M1 à M6. Dans chacun de ces 6 flacons, on ajoute 200 mL d'eau pour préparation injectable. On obtient ainsi 6 plasmas reconstitués PR1 à PR6.

Après reconstitution, le produit obtenu doit répondre aux exigences suivantes:
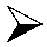 temps de reconstitution inférieur à 6 minutes;
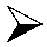 concentration en facteur VIII supérieure ou égale à 0,5 UI/L;
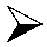 absence d'hémolysines anti-A et anti-B;
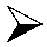 titre en agglutinines anti A et anti B inférieur à 64;
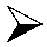 absence d'agglutinines irrégulières; et
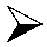 concentration en protéines supérieure ou égale à 50 g/L.

La composition et les caractéristiques des plasmas ainsi reconstitués sont détaillées dans le tableau ci après:

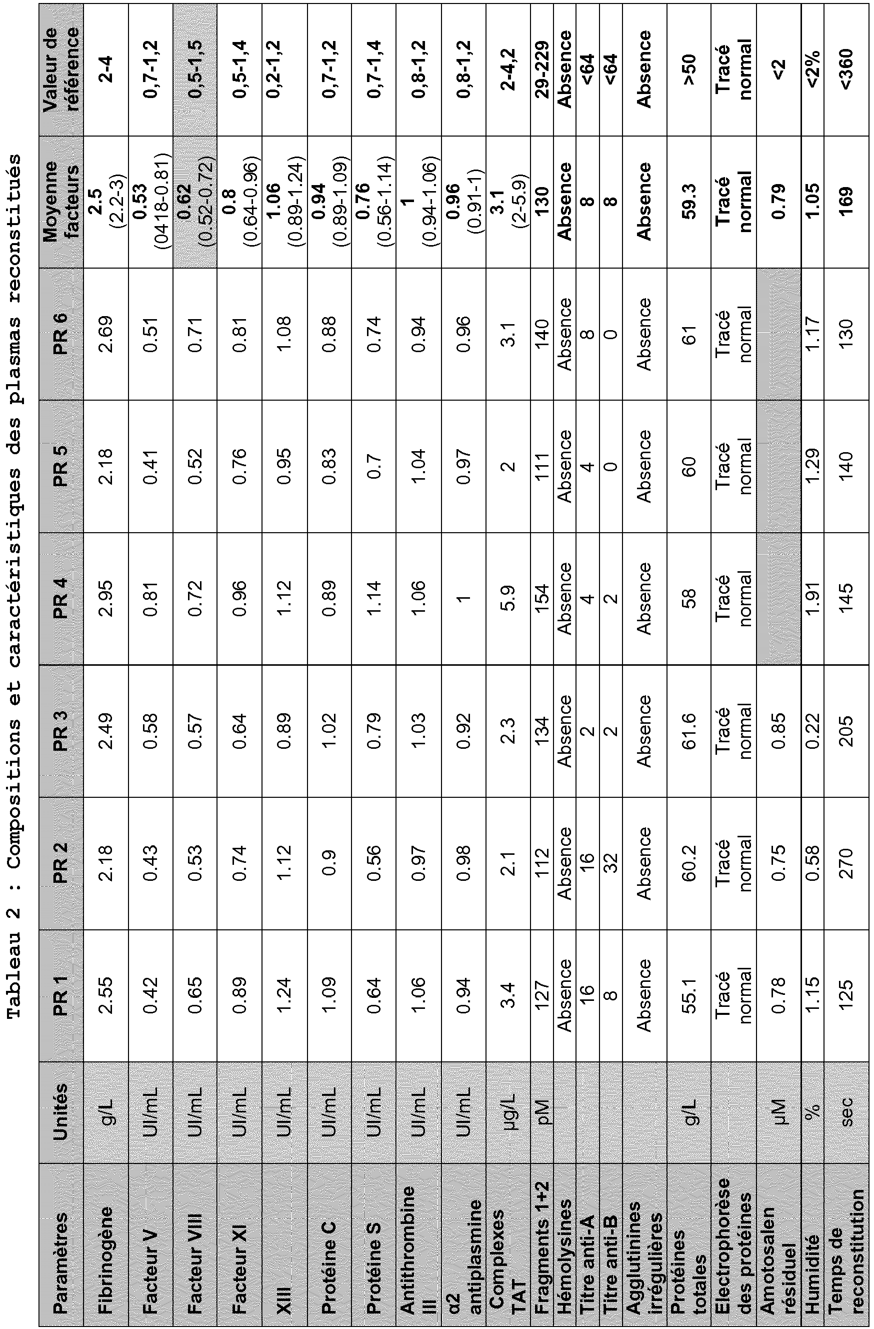

Les critères mesurés sont conformes aux exigences réglementaires. Le plasma obtenu selon le procédé de l'invention est donc adapté à une utilisation thérapeutique.

### Exemple 3: Effet de la lyophilisation sur les coagulopathies et les hémorragies

Les inventeurs ont administré le plasma lyophilisé reconstitué selon l'invention sur le théâtre d'actions militaires, chez des soldats ayant subi de graves traumatismes tels que des blessures infligées par armes à feu, après explosions, ou encore après collisions.

### Matériels et Méthode

Les soldats perfusés ont subi une analyse sanguine et une analyse de l'hémostase avant et après la perfusion du plasma lyophilisé reconstitué selon l'invention.

Les variables ainsi récoltées ont été analysées selon les techniques statistiques classiques, basée sur le test de Student, le test de Wilcoxon ou encore le chi2.

87 soldats on été perfusé à l'aide du plasma lyophilisé selon l'invention, dont 32 victimes de blessures par balles, 22 de polytraumatismes, 10 d'explosions, 7 de traumatismes crâniens et enfin 4 d'autres types de blessures.

Les données liées à cette transfusion sont récapitulées dans le tableau ci-dessous:

**Tableau 3 : Caractéristiques sanguines des soldats transfusés**

| **Caractéristiques avant administration du sang** | **Médiane** | **Rang** | **Pourcentage de patient (%)** |
|---|---|---|---|
| Erythrocytes (unité) | 3 | 1-13 | 32 |
| Sang total (unité) | 4 | 1-7 | 5 |
| Cristalloïde (L) | 1 | 0,2-5 | 56 |
| Colloïde (mL) | 500 | 100-8000 | 15 |
| Facteur VIIa (mg) | 2 | 1-7 | 9 |
| Fibrinogène (g) | 1,5 | 1-3 | 6 |

### Résultat

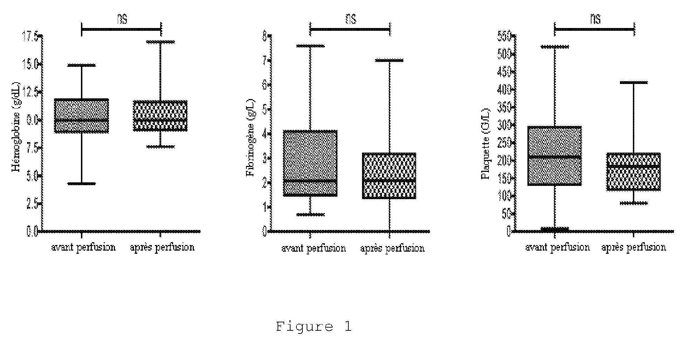

Les résultats sont présentés à la figure 1.

Les différences dans les taux d'hémoglobine, de plaquettes et de fibrinogène avant et après transfusion s'avèrent non significatifs. Aussi, les inventeurs ont montré que le niveau de facteurs sanguins se trouve non affecté et que l'utilisation du plasma de l'invention s'avère sans risque sur la santé du soldat ayant subi la perfusion dudit plasma.

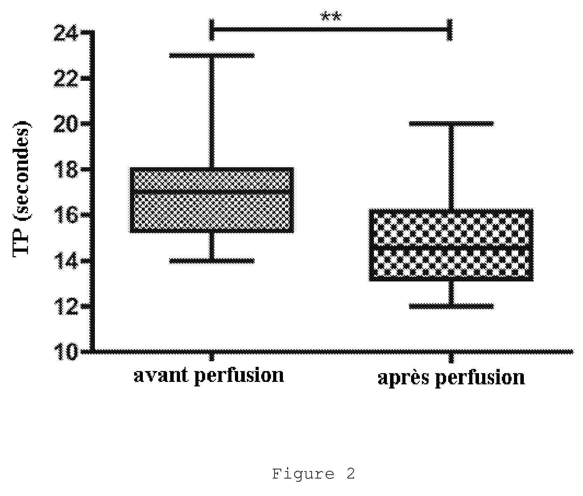

D'autre part, les inventeurs ont réussi à montrer que le plasma lyophilisé selon l'invention permet le contrôle des coagulopathies dans les blessures de guerre. En outre, les inventeurs ont entre mis en évidence le fait que la perfusion de plasma lyophilisé sanguin de l'invention permet la réduction du taux de prothrombine, comme le montre la figure 2. Ces résultats indiquent que le plasma de l'invention est efficace pour le traitement des hémorragies.

Enfin, les résultats montrent que le plasma lyophilisé selon l'invention permet d'obtenir d'excellents résultats avec l'avantage majeur de pouvoir être reconstitué en moins de 6 minutes et de fournir une quantité de 210 mL de plasma prêt à l'emploi. Ces résultats sont d'ailleurs comparables à ceux susceptibles d'être obtenus lors de perfusion avec du plasma frais congelé.

### Exemple 4: Utilisation du plasma lyophilisé selon l'invention dans le traitement d'hémorragie massive

De nombreuses études montrent que la perfusion de plasma de manière conséquente et précoce améliore considérablement la survie des soldats lors d'actions militaires. 63 soldats ont été perfusés d'une à neuf poches de plasma conforme à l'invention.

Les inventeurs ont analysé leur sang avant et après la perfusion dudit plasma. Les résultats montrent que le taux de prothrombine est passé de 17 sec avant ladite perfusion à 15,6 secondes après ladite perfusion.

Cette diminution indique une amélioration de l'hémostase, amélioration qui peut s'avérer cruciale préalablement à une intervention chirurgicale.

L'utilisation du plasma lyophilisé reconstitué selon l'invention n'a montré aucun effet indésirable.

### Exemple 5: Effet de la lyophilisation sur les capacités hémostatiques globales du plasma lyophilisé reconstitué selon l'invention

Les inventeurs ont étudié l'effet de la lyophilisation sur la qualité du plasma obtenu et son effet sur l'hémostase.

### Matériels et méthode

Les inventeurs ont mis en oeuvre 24 batch avant et après lyophilisation.

Les inventeurs ont déterminé le niveau du fibrinogène ainsi que des facteurs de coagulation V, VIII, XI et XIII à l'aide d'essais colorimétriques et chronométriques. Un test de génération de thrombine a également été mis en oeuvre. Puis, après dilution du plasma lyophilisé et d'un contrôle standardisé, une thromboélastographie a été effectuée.

Enfin, le sang de 17 donneurs sains a été dilué jusqu'à 60%:
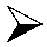 60% de ringer lactate, ou
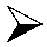 30% de ringer lactate + 30% de plasma lyophilisé avant lyophilisation, ou
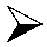 30% de ringer lactate + 30% FDP après lyophilisation.
Une thromboélastographie enfin a été mise en oeuvre.

### Résultat

Les résultats obtenus après lyophilisation sont résumé ci après:
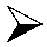 une diminution d'environ 22 à 26% dans l'activité du facteur VIII et le factor V (0,6 UI/mL ± 0,1);
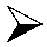 une diminution d'environ 10% dans le niveau de la protéine S (0,8 UI/mL ± 0,2);
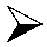 aucune diminution de l'activité du fibrinogène (2,4 g/L ± 0,2), du facteur X (0,8 UI/mL ± 0,1), du facteur XIII (1 UI/mL ± 0,1), de la protéine C (0,9 UI/mL ± 0,1), et de l'alpha2-antiplasmine (1 UI/mL ± 0,1);
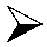 pas d'altération de la génération de thrombine; et
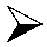 pas d'activation des facteurs de coagulation.

Les inventeurs ont également montré qu'après dilution, la lyophilisation n'induit pas de modification dans les paramètres du thromboélastogramme.

Ces résultats quantitatifs et qualitatifs indiquent que les capacités globales d'hémostase du plasma lyophilisé sont conservés, en comparaison avec le plasma non lyophilisé.

## Revendications

1. Procédé de préparation de plasma lyophilisé viro-atténué comprenant les étapes suivantes:
A) la sélection de plasmas unitaires déleucocytés et viro-atténué par action d'un agent photochimique, lesdits plasmas étant obtenus à partir d'au plus dix individus donneurs différents et lesdits donneurs présentant un bilan d'hémostase normal, un taux en facteur VIII supérieur à 0,9 UI/mL et étant choisis parmi les donneurs masculins ou les donneurs exempts d'anticorps anti-HLA;
B) le mélange des plasmas unitaires sélectionnés; et
C) la lyophilisation dudit mélange de plasmas.

2. Procédé selon la revendication 1, **caractérisé en ce que** ledit agent photochimique est choisi parmi l'amotosalen, le bleu de méthylène et le riboflavin.

3. Procédé selon la revendication 2 ou 3, **caractérisé en ce que** ledit agent photochimique est l'amotosalen.

## Patentansprüche

1. Verfahren für die Aufbereitung gefriergetrockneten virus-attenuierten Plasmas, welches die folgenden Schritte umfasst:
A) Auswahl einheitlicher, leukozytendepletierter und virus-attenuierter Plasmen durch Einsatz eines photochemischen Mittels, wobei die Plasmen von höchstens zehn verschiedenen Spendern erhalten werden und die Spender ein normales Hämostasebild und einen Faktor VIII-Wert größer 0,9 UI/ml aufweisen und aus männlichen Spendern oder Spendern ohne HLA-Antikörper gewählt werden;
B) Mischung der gewählten einheitlichen Plasmen; und
C) Gefriertrocknung der Plasmen-Mischung.

2. Verfahren nach Anspruch 1, **dadurch gekennzeichnet, dass** das photochemische Mittel gewählt wird aus Amotosalen, Methylenblau und Riboflavin.

3. Verfahren nach Anspruch 2 oder 3, **dadurch gekennzeichnet, dass** das photochemische Mittel Amotosalen ist.

## Claims

1. A process for preparing virus-attenuated, lyophilised plasma, comprising the following steps:
A) selecting leuko-depleted and virus-attenuated unit plasmas by the action of a photochemical agent, said plasmas being obtained from at most ten different donor individuals and said donors having normal haemostasis test results, a factor VIII level greater than 0.9 IU/mL and being selected from male donors or donors free of anti-HLA antibodies;
B) mixing the selected unit plasmas; and
C) lyophilising said mixture of plasmas.

2. The process as claimed in claim 1, **characterised in that** said photochemical agent is selected from amotosalen, methylene blue and riboflavin.

3. The process as claimed in claim 2 or 3, **characterised in that** said photochemical agent is amotosalen.
